Europäisches Patentamt

(19) European Patent Office

Office européen des brevets

(11) Publication number: **0 179 536**
A2

(12) **EUROPEAN PATENT APPLICATION**

(21) Application number: 85201718.5

(22) Date of filing: 22.10.85

(51) Int. Cl.⁴: **A 61 F 2/00**
A 61 N 1/378, H 04 R 25/00

(30) Priority: 22.10.84 AU 67767/84

(43) Date of publication of application:
30.04.86 Bulletin 86/18

(84) Designated Contracting States:
DE FR GB

(71) Applicant: Cochlear Pty., Ltd.
P.O. Box 629
Lane Cove New South Wales 2066(AU)

(72) Inventor: Harrison, James Mark
266 Nell Street
Watsonia Victoria 3078(AU)

(72) Inventor: Seligman, Peter Misha
107 Fowlner Street
Essendon Victoria 3040(AU)

(74) Representative: Noz, Franciscus Xaverius, Ir. et al,
Boschdijk 155 P.O. Box 645
NL-5600 AP Eindhoven(NL)

(54) Improvements in power transfer for implanted prostheses.

(57) Apparatus for improving the coupling of an external inductive transmitting coil to an internal inductive receiving coil to transmit power and/or data to said receiving coil from said transmitting coil whereby a coupling coil inductively coupled to said transmitting coil to increase the Q factor between said transmitting coil and said receiving coil.

IMPROVED DRIVER
FIG. 4

-1-

IMPROVEMENTS IN POWER TRANSFER FOR IMPLANTED PROSTHESES

This invention relates to improvements in systems for electromagnetically transferring power and/or data, especially, but not exclusively, in implanted prostheses.

Many electronic prostheses implanted in the body do not have a self contained power source such as a battery, but rely on an external source of power located outside the body. In addition to this, some prostheses also require an external source of data, linked via externally worn electronics to a microphone.

A cochlear implant is a device which restores hearing sensations for profoundly deaf people by direct electrical stimulation of the nerve fibres in the inner ear, or cochlea. Power and data is electromagnetically coupled to an implanted receiver coil from an external transmitting coil worn over the site of the implant.

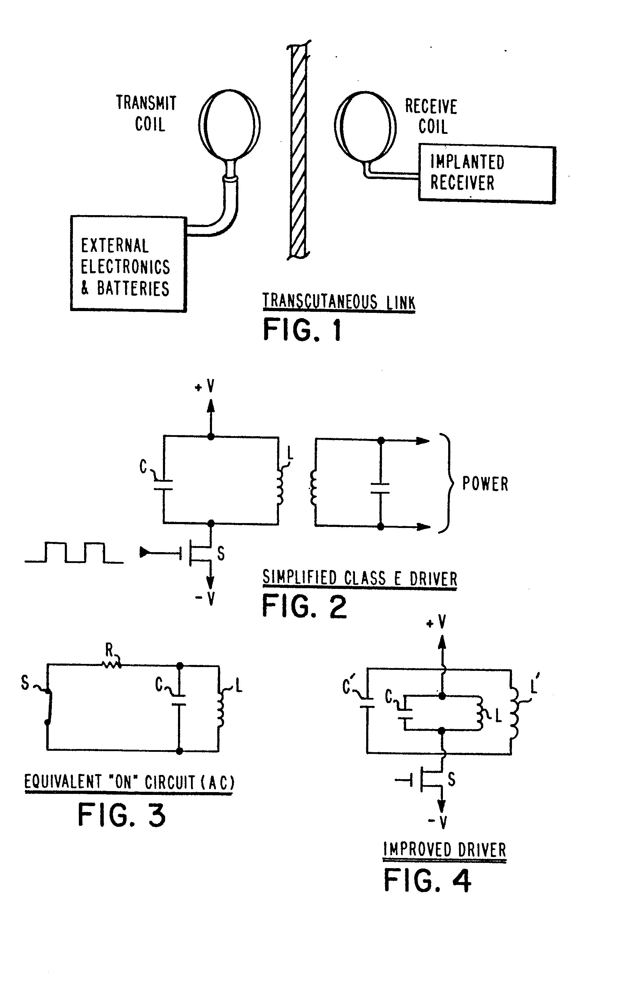

A radio frequency induction link of this type is depicted in Fig. 1. The power transfer efficiency of this link is related both to the coupling co-efficient, k, between the two coils and the product of their Q or quality factors. Thus any mechanism to increase these parameters will result in a higher transfer efficiency.

The class E tuned amplifier is a simple and efficient driver which is often used in transcutaneous power and data transmitters for implanted electronic prostheses. Such a driver is depicted in Fig. 2. The operation of this circuit is as follows. After the transistor switch S is turned on the current in the inductor L builds up and the switch S turns off. The energy from the inductor is then transferred to the capacitor C and the tuned circuit formed by L and C rings for one half of one cycle. The voltage on the inductor L reaches zero again and simultaneously, the switch S is turned on again. The cycle is then repeated.

It will be apparent that if the switch S is imperfect, during the period T(on) the transmitting tuned circuit will appear as shown in Fig. 3, and thus energy will be dissipated in the equivalent resistance, R, leading to inefficiencies.

It will also be apparent that during this interval, the Q of the transmitter tuned circuit is reduced by the resistance of the switch, thus limiting the efficiency of the power transfer. While the switch is on, the transmitter tuned circuit virtually ceases to exist and is replaced by a shortcircuited coil.

It is an object of the present invention to provide an improved transfer system whereby the above disadvantages are at least ameliorated.

In its broadest form, the invention provides a system for electromagnetically transferring power and/or data comprising a primary transmitter circuit adapted to transfer said power and/or data to a secondary receiver circuit, and a tertiary circuit electromagnetically coupled with said primary circuit and tuned to increase the effective Q of the transmitter circuit.

The primary transmitter circuit preferably includes a coil which is adapted to be inductively coupled with the secondary receiving circuit which also includes a coil, said tertiary circuit including a tuned coil which is loosely coupled with said primary coil. The primary and secondary coil are preferably located in close proximity to each other to achieve the necessary inductive coupling.

In an alternative embodiment, said tertiary coil is positioned closely adjacent to said secondary coil while still being loosely coupled with said primary coil.

More specifically, the present invention provides an improvement in an auditory prosthesis including at least one external coil electromagentically coupled to at least one internal coil for the purposes of transmitting power and/or information through the skin, said improvement comprising at least one tertiary tuned coil located adjacent to an electromagnetically coupled to said external and internal coils.

Thus, we have found that by placing a loosely coupled, tuned, tertiary coil near the transmitter coil (shown as L' and C' in Fig. 4) the effective Q of the transmitter circuit (modelled as a lumped, linear circuit) is increased with a resultant increase in transfer efficiency.

It should be noted that the effect of a nearby shortcircuited inductor on a tuned circuit is to change the frequency of resonance. This must be taken into account in tuning the tertiary tuned circuit. It will also be noted that the improvement in efficiency is not restricted to systems using the Class E driver stage, but is also applicable to any other practically realisable output driver.

Further it can be seen that the improvement may be realised by using another component such as an inductor or capacitor, directly connected to the tertiary tuned coil, rather than using the method of coupling by mutual inductance. This component could itself form part of the tertiary tuned circuit but need not be physically close to the transmitter coil.

In the cochlear implant system previously described in a copending application (US Serial No. 06/483,806 filed April 11, 1983, "Cochlear Implant System for an Auditory Prosthesis"), the transmitter coil consisted of approximately 8 turns of enamelled copper wire with a diameter of approximately 30 mm, tuned with a single ceramic dielectric capacitor of above 100 pF. The improvement in this case was to put the tertiary tuned coil consisting of 8 turns of enamelled copper wire with a diameter of 40 mm, tuned with a ceramic capacitor of about 540 pF, and coaxial to and coplanar with the transmitter coil. This resulted in an improvement of almost 100% in operating range of the system for approximately 30% less power consumption.

In another embodiment the tertiary coil is mounted coaxially to but inside the primary coil, with suitable adjustments to the inductance and capacitance.

We have discovered that it is not necessary for the tertiary coil to be coaxial, or coplanar with the other coils, but merely coupled with it.

It will be apparent to those skilled in the art that this invention has application in areas other than cochlear prostheses such as visual prostheses, cerebellar stimulators, pain control devices etc., or any implanted electronic device where it is required to couple power through the skin. The improvement also has application with prostheses

0179536

where more than one coil is required to transmit to at least one internal coil. The idea is also useful where it is necessary to couple power and/or information between electronic devices through a wall or membrane, such as in chemical engineering processes.

Modifications and adaptations may be made to the above described without departing from the spirit and scope of this invention which includes every novel feature and combination of features disclosed herein.

-1-

CLAIMS

We claim:

1.     Apparatus for improving the coupling of an external inductive transmitting coil to an internal inductive receiving coil to transmit power and/or data to said receiving coil from said transmitting coil comprising:

    a coupling coil inductively coupled to said transmitting coil to increase the Q factor between said transmitting coil and said receiving coil.

2.     Apparatus in accordance with Claim 1 in which said coupling coil is coplanar with said transmitting coil.

3.     Apparatus in accordance with Claim 1 in which said coupling coil is concentric with said transmitting coil.

4.     Apparatus in accordance with Claim 3 in which said coupling coil is disposed outside said transmitting coil.

5.     Apparatus in accordance with Claim 3 in which said coupling coil is disposed inside said transmitting coil.

6.     Apparatus in accordance with Claim 1 including capacitance means connected to each of said transmitting coil and said coupling coil to form LC circuits therewith.

7.     Apparatus in accordance with Claim 1 in which said internal receiving coil is part of an implanted prosthesis and said external transmitting coil includes means for coupling power and/or data to said implanted prosthesis.

0179536

TRANSMIT COIL

RECEIVE COIL

IMPLANTED RECEIVER

EXTERNAL ELECTRONICS & BATTERIES

TRANSCUTANEOUS LINK

FIG. 1

+V

C        L

POWER

S

-V

SIMPLIFIED CLASS E DRIVER

FIG. 2

R

S        C        L

EQUIVALENT "ON" CIRCUIT (AC)

FIG. 3

+V

C'        C        L        L'

S

-V

IMPROVED DRIVER

FIG. 4